Europäisches Patentamt

European Patent Office

Office européen des brevets

(19)

(11) Veröffentlichungsnummer: **0 113 854**
**B1**

# (12) EUROPÄISCHE PATENTSCHRIFT

(45) Veröffentlichungstag der Patentschrift:
08.04.87

(21) Anmeldenummer: 83112316.1

(22) Anmeldetag: 07.12.83

(51) Int. Cl.⁴: **A 61 N 1/362**

(54) **Bifokaler Herzschrittmacher mit zwei unipolaren Elektroden.**

(30) Priorität: 21.12.82 DE 3247264

(43) Veröffentlichungstag der Anmeldung:
25.07.84 Patentblatt 84/30

(45) Bekanntmachung des Hinweises auf die Patenterteilung:
08.04.87 Patentblatt 87/15

(84) Benannte Vertragsstaaten:
DE FR NL

(56) Entgegenhaltungen:
EP-A-0 033 242
WO-A-82/03785
DE-A-2 628 629
FR-A-2 524 807
US-A-3 903 897
US-A-3 945 374
US-A-4 170 227
US-A-4 263 919
US-A-4 365 639

(73) Patentinhaber: **Siemens Aktiengesellschaft Berlin und München, Wittelsbacherplatz 2, D-8000 München 2 (DE)**

(72) Erfinder: **Strandberg, Hans, Landsvägen 51, S-17 236 Sundbyberg (SE)**
Erfinder: **Andersen, Hans, Strandliden 19, S-16 238 Vällingby (SE)**

EP 0 113 854 B1

## Beschreibung

Die Erfindung betrifft einen bifokalen Herzschrittmacher mit zwei unipolaren Elektroden, mindestens einer im Abstand zu diesen liegenden indifferenten Elektrode, je einem Verstärker, gegebenenfalls Filtern und mit nachfolgendem Detektor per unipolarer Elektrode sowie Mitteln zum Steuern von Stimulierungsimpulsen.

Ein derartiger Herzschrittmacher ist bereits aus dem Dokument DE-A- 8 203 785 bekannt.

Bei einem derartigen Herzschrittmacher können beispielsweise die Elektroden in der Vorkammer und in der Herzkammer plaziert sein. Die indifferente Elektrode wird beispielsweise durch die Kapsel des Herzschrittmachers gebildet. Ein derartiges Elektrodensystem mit unipolaren Elektroden hat sehr gute Stimulations- und auch Detektionseigenschaften. Hingegen besteht bei einem derartigen System die Gefahr, dass durch Muskelzuckungen hervorgerufene Störsignale oder von ausserhalb des Körpers herstammende Störsignale das einwandfreie Funktionieren eines derartigen Herzschrittmachers beeinträchtigen. Um z.B. zu verhindern, dass die durch Muskelzuckungen in der Nähe der Herzschrittmacherkapsel hervorgerufenen Signale von dieser als Herzsignale detektiert werden, ist es beispielsweise bekannt, die Kapsel grösstenteils zu isolieren und nur einen leitenden Bereich freizulassen, den man dann bei der Implantation von dem Muskelgewebe abwendet. Dabei verkleinert sich aber die leitfähige Oberfläche und es können Polarisationsprobleme entstehen.

Der vorliegenden Erfindung liegt die Aufgabe zugrunde, auf einfache und sichere Weise Störsignale beliebiger Art zu unterdrücken, ohne dabei die guten Stimulations- und/oder Detektionseigenschaften des Herzschrittmachers negativ zu beeinflussen.

Diese Aufgabe wird erfindungsgemäss dadurch gelöst, dass im Herzschrittmacher eine Signalbehandlungseinrichtung vorgesehen ist, auf die die Ausgangssignale der beiden Detektoren gegeben sind und die beim annähernd gleichzeitigen Vorliegen eines Signals von beiden Detektoren die Abgabe eines Ausgangssignals an die Mittel zum Steuern der Stimulierungsimpulse unterdrückt. Die Erfindung geht dabei von der Erkenntnis aus, dass die natürlichen Herzsignale praktisch nur von jeweils einer Elektrode detektiert werden. So erzeugt ein QRS-Komplex ein Ausgangssignal des an die in der Herzkammer angeordneten Elektrode angeschlossenen Detektors. Die in der Vorkammer angeordnete Elektrode registriert demgegenüber nur ein vernachlässigbar kleines Signal. Bei einem Störsignal liegen die Verhältnisse anders. In einem solchen Fall registrieren beide Elektroden annähernd gleichwertige Signale. Das Auftreten gleicher Signale an beiden Elektroden ist damit ein sicherer Hinweis für das Vorliegen einer Störung.

In Weiterbildung der Erfindung ist vorgesehen, dass ein weiterer Verstärker mit nachfolgendem Detektor vorgesehen ist, dem die Signale der beiden Elektroden, gegebenenfalls nach Verstärkung, zugeführt sind, und dass die Ausgangssignale aller drei Detektoren auf die Signalbehandlungseinrichtung gegeben sind. Liegen annähernd gleichzeitig Signale von den beiden den einzelnen Elektroden zugeordneten Detektoren vor, unterdrückt die Signalbehandlungseinrichtung wiederum die Abgabe von Ausgangimpulsen dieser Detektoren an die Mittel zur Beeinflussung der Stimulierungsimpulse. Die Signalbehandlungseinrichtung kann einfach und kostengünstig aus logischen Gliedern aufgebaut sein. Sämtliche Komponenten für die Verstärker, Filter, Detektoren und auch die Signalbehandlungseinrichtungen können sowohl in Analog- als auch in Digitaltechnik ausgeführt sein.

Anhand dreier Figuren werden im folgenden Ausführungsbeispiele der Erfindung näher beschrieben und erläutert. Dabei zeigt

Fig.1 ein Blockschaltbild des erfindungsgemässen Herzschrittmachers mit Störsignaldetektor

Fig.2 die von der in der Elektrode in der Vorkammer bzw. der in der Herzkammer möglicherweise empfangenen Signale und

Fig.3 eine mögliche Ausführungsform der Signalbehandlungseinrichtung.

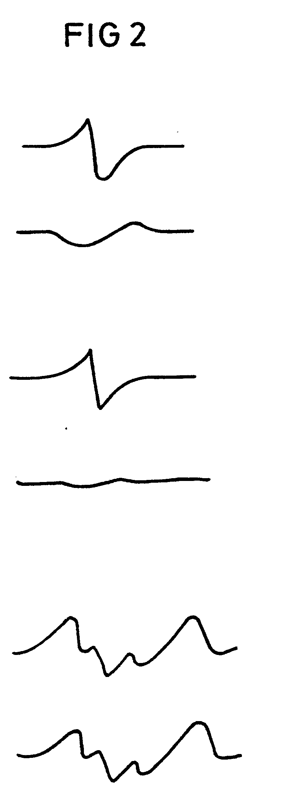

Die Fig.2 zeigt untereinander drei Paare von Signalen, wobei jeweils das oberste jeden Paares das von der Herzkammerelektrode aufgenommene Signal und das untere das von der Vorkammerelektrode aufgenommene Signal darstellt. Für das erste Paar ist ein QRS-Komplex in der Herzkammer als Signalquelle angenommen. Die Herzkammerelektrode zeigt den typischen Signalverlauf dafür. Die Vorkammerelektrode zeigt wegen des verhältnismässig grossen Abstandes zur Signalquelle ein nahezu vernachlässigbar kleines Signal. Im mittleren Signalpaar ist als Signalquelle eine Vorkammeraktivität (P-Welle) angenommen. In diesem Falle zeigt die Vorkammerelektrode ein grosses Signal, die Herzkammerelektrode praktisch kein Signal.

Für das dritte Signalpaar ist ein Störsignal als Signalquelle vorausgesetzt. In diesem Fall zeigen beide Elektroden etwa gleich grosse und sich auch in der Form entsprechende Signalverläufe.

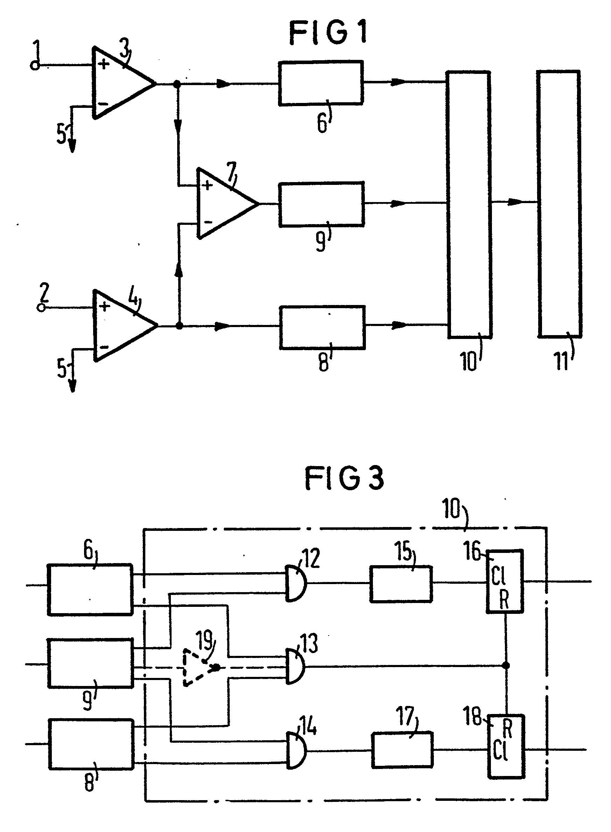

In Fig.1 ist ein Blockschaltbild eines Herzschrittmachers gemäss der Erfindung dargestellt. Dabei sind mit 1 bzw. 2 die Anschlüsse für die Vorkammer- bzw. Herzkammerelektrode bezeichnet. Diese sind jeweils auf einen Eingang eines Verstärkers 3 bzw. 4 gegeben. Als indifferente Elektrode für beide - sowohl die Vorkammer- als auch Herzkammerelektrode - dient beispielsweise die Kapselung des Herzschrittmachers. Die indifferente Elektrode 5 ist an den jeweils anderen Eingang der beiden Verstärker 3 und 4 angeschlossen. Ohne darauf näher einzugehen sei hier angenommen, dass die Verstärker gleichzeitig auch noch Filter enthalten können.

Das Ausgangssignal des Verstärkers 3 ist auf einen ersten Detektor 6 und gleichzeitig auf einen Eingang eines Differentialverstärkers 7 gegeben. Das Ausgangssignal des Verstärkers 4 ist auf einen weiteren Detektor 8 und gleichzeitig auf den anderen Eingang des Differentialverstärkers 7 gegeben. Das Ausgangssignal des Differentialverstärkers 7 ist einem zusätzlichen Detektor 9 zugeführt. Die drei Detektorausgangssignale sind auf eine Signalbehandlungseinrichtung 10 gegeben, an die sich die restliche Herzschrittmacherelektronik anschliesst, die im vorliegenden Ausführungsbeispiel als Block 11 dargestellt ist und auf die hier nicht näher eingegangen zu werden braucht. Diese restliche Herzschrittmacherelektronik kann in der für bifokale Herzschrittmacher üblichen Weise aufgebaut sein.

Ein Blick auf die in Fig.2 dargestellten Signalverläufe verdeutlicht, dass Detektor 6 jeweils ein Ausgangssignal liefert, wenn die Vorkammerelektrode ein Signal empfängt. Entsprechend liefert Detektor 8 ein Ausgangssignal, wenn die Herzkammerelektrode ein Signal empfängt. Werden diese Signale durch echte Herzaktivitäten hervorgerufen, so ergibt sich jedesmal auch ein Ausgangssignal des Differenzverstärkers 7 und damit ein Ausgangssignal des zusätzlichen Detektors 9. Liegt hingegen der Fall einer externen Störung oder generell eines Störsignales vor, so empfangen beide Elektroden gleichzeitig das Signal, so dass zwar die Detektoren 6 und 8 ein Ausgangssignal liefern, der Detektor 9 jedoch nicht, da der Differenzverstärker 7 auf beiden Eingängen gleiche Signale empfängt und damit kein Ausgangssignal erzeugt.

Wie die Signalbehandlungseinrichtung 10 aus den Ausgangssignalen der drei Detektoren 6, 8 und 9 ermittelt, ob eine echte Herzaktivität oder eine Störung vorliegt, lässt sich am einfachsten anhand einer Tabelle darstellen:

| Signalart | Ausgangssignal des Detektors |
|---|---|
| P-Welle | 6 und 9 |
| QRS-Komplex | 8 und 9 |
| Störsignal | 6 und 8 |

Wie aus dieser Tabelle ersichtlich, liegt den empfangenen Signalen eine echte Herzaktivität zugrunde, wenn einer der den Elektroden direkt zugeordnete Detektoren und der zusätzlich über den Differenzverstärker angeschlossene Detektor ein Ausgangssignal liefert. Geben hingegen beide den einzelnen Elektroden zugeordnete Detektoren ein Ausgangssignal, so ist das das Kriterium für das Vorliegen einer Störung. In diesem Falle unterdrückt die Signalbehandlungseinrichtung die Weiterleitung eines Signales zur Steuerung der Stimulierungsimpulse.

In der folgenden Fig.3 ist eine mögliche Ausführungsform der Signalbehandlungseinrichtung 10 wiederum als Blockschaltbild dargestellt. Ausserdem sind zusätzlich die Detektoren 6, 8 und 9 dargestellt. Die Signalbehandlungseinrichtung weist drei UND-Glieder 12 bis 14 auf, wobei auf das UND-Glied 12 die Ausgangssignale der Detektoren 6 und 9, auf das UND-Glied 13 die Ausgangssignale der Detektoren 6, 8 und gegebenenfalls über einen Inverter 19 das des Detektors 9 und auf das UND-Glied 14 die Ausgangssignale der Detektoren 8 und 9 gegeben sind. Das Ausgangssignal des UND-Gliedes 12 wird über eine Verzögerungsstufe 15 dem Steuereingang einer Kippstufe 16 zugeführt. Entsprechend wird das Ausgangssignal des UND-Gliedes 14 über eine weitere Verzögerungsstufe 17 einer weiteren Kippstufe 18 zugeführt. Das Ausgangssignal des UND-Gliedes 13 ist gleichzeitig auf die Rückstelleingänge dieser beiden Kippstufen 16 bzw. 18 gegeben. Zusätzlich kann, wie gestrichelt angedeutet, das Ausgangssignal des Detektors 9 über einen Inverter 19 auf einen weiteren Eingang des UND-Gliedes 13 gegeben sein. Damit wird die Sicherheit, dass natürliche Herzsignale auch als solche behandelt werden und nicht u.U. als Störsignale, erhöht.

Die Wirkungsweise dieser Signalbehandlungseinrichtung ist folgende:

Liegt eine Vorkammeraktivität (P-Welle) dem Signal zugrunde, so liefern die Detektoren 6 und 9 je ein Ausgangssignal. Das UND-Glied 12 steuert durch und setzt nach einer kurzen Verzögerung von beispielsweise einigen ms den Ausgang der Kippstufe 16 hoch. Dieses Signal zeigt an, dass ein P-Welle vorgelegen hat. Liegt hingegen eine Störung vor, so steuert lediglich die UND-Stufe 13 durch und beide Kippstufen 16 bzw. 18 werden zurückgesetzt.

**Patentansprüche**

1. Bifokaler Herzschrittmacher mit zwei unipolaren Elektroden, mindestens einer im Abstand zu diesen liegenden indifferenten Elektrode, je einem Verstärker, gegebenenfalls Filtern und mit nachfolgendem Detektor per unipolarer Elektrode sowie Mitteln zum Steuern von Stimulierungsimpulsen, dadurch gekennzeichnet, dass eine Signalbehandlungseinrichtung (10) vorgesehen ist, auf die mindestens die Ausgangssignale der beiden Detektoren (6, 8) gegeben sind und die bei annähernd gleichzeitigem Vorliegen eines Signals von beiden Detektoren (6, 8) die Abgabe eines Ausgangssignales an die Mittel (11) zum Steuern der Stimulierungsimpulse unterdrückt.

2. Herzschrittmacher nach Anspruch 1, dadurch gekennzeichnet, dass ein weiterer Verstärker (7) mit nachfolgendem Detektor (9) vorgesehen ist, dem die Signale der beiden Elektroden (1, 2), gegebenenfalls nach Verstärkung, zugeführt sind, und dass die Ausgangssignale aller drei Detektoren (6, 8, 9) auf die Signalbehandlungseinrichtung (10) gegeben sind, die beim annähernd gleichzeitigen Vorliegen eines Signales von den beiden den einzelnen Elektroden zugeordneten Detektoren (6, 8) die Abgabe eines Ausgangssignales an die Mittel (11) zum Steuern der Stimulierungsimpulse unterdrückt.

3. Herzschrittmacher nach Anspruch 1 oder 2, dadurch gekennzeichnet, dass die Signalbehandlungseinrichtung (10) im wesentlichen aus logischen Gliedern (12-14) aufgebaut ist.

4. Herzschrittmacher nach einem der Ansprüche 1 bis 3, dadurch gekennzeichnet, dass die Verstärker (3, 4, 7), Filter, Detektoren (6, 8, 9) und die Signalbehandlungseinrichtung (10) zumindest teilweise in Digitaltechnik ausgeführt sind.

## Claims

1. A bifocal heart-pacemaker having two unipolar electrodes, at least one indifferent electrode arranged at an interval from the two unipolar electrodes, a respective amplifier for each of the two unipolar electrodes, with appropriate filters and a respective detector, and means for controlling stimulating pulses, characterised in that a signal-processing device (10) is provided which is supplied at least with the output signals of the two detectors (6, 8) and which suppresses the emission of an output signal to the means (11) which control the stimulating pulses when a signal occurs approximately simultaneously from both detectors (6, 8).

2. A heart-pacemaker as claimed in Claim 1, characterised in that a further amplifier (7) is provided which is connected at its output end to a detector (9) and supplied with the signals of the two electrodes (1, 2), possibly following amplification, and that the output signals of all three detectors (6, 8, 9) are fed to the signal-processing device (10) which suppresses the emission of an output signal to the means (11) which control the stimulating pulses when a signal occurs approximately simultaneously from the two detectors (6, 8) assigned to the individual electrodes.

3. A heart-pacemaker as claimed in Claim 1 or 2, characterised in that the signal-processing device (10) fundamentally consists of logic elements (12-14).

4. A heart-pacemaker as claimed in one of Claims 1 to 3, characterised in that the amplifiers (3, 4, 7), filters, detectors (6, 8, 9) and the signal-processing device (10) are constructed at least partially in digital technology.

## Revendications

1. Stimulateur cardiaque bifocal comportant deux électrodes unipolaires, au moins une électrode indifférente située à distance de ces électrodes, des amplificateurs respectifs, éventuellement des filtres, et comportant un détecteur branché en aval pour chaque électrode unipolaire, ainsi que des moyens pour la commande d'impulsions de stimulation, caractérisé par le fait qu'il est prévu un dispositif (10) de traitement des signaux, auquel sont envoyés au moins les signaux de sortie des deux détecteurs (6, 8) et qui, lors de la présence approximativement simultanée de signaux provenant des deux détecteurs (6, 8), interrompt la délivrance du signal de sortie aux moyens (11) de commande des impulsions de stimulation.

2. Stimulateur cardiaque suivant la revendication 1, caractérisé par le fait qu'il est prévu un autre amplificateur (7), en aval duquel est branché un détecteur (9) et auquel sont envoyés, éventuellement après amplification, les signaux des deux électrodes (1, 2) et que les signaux de sortie de l'ensemble des trois détecteurs (6, 8, 9) sont envoyés au dispositif (10) de traitement des signaux qui, lors de la présence approximativement simultanée de signaux provenant des deux détecteurs (6, 8) associés aux différentes électrodes, interrompt la délivrance d'un signal de sortie aux moyens (11) de commande des impulsions de stimulation.

3. Stimulateur cardiaque suivant la revendication 1 ou 2, caractérisé par le fait que le dispositif (10) de traitement des signaux est constitué essentiellement par des circuits logiques (12-14).

4. Stimulateur cardiaque suivant l'une des revendications 1 à 3, caractérisé par le fait que les amplificateurs (3, 4, 7), les filtres, les détecteurs (6, 8, 9) et le dispositif (10) de traitement des signaux sont réalisés au moins partiellement selon la technique numérique.

# FIG 1

# FIG 3

0 113 854

FIG 2